# EUROPEAN PATENT SPECIFICATION

(11) **EP 1 335 743 B1**
(45) Date of publication and mention of the grant of the patent: **23.12.2009**
(21) Application number: 01981902.8
(22) Date of filing: 02.11.2001
(51) Int. Cl.: A61K 38/08, A61K 38/32, C07K 7/06, C07K 14/66

(54) **INHIBITION OR REVERSAL OF SKIN AGING BY ACTIN-SEQUESTERING PEPTIDES**
HEMMUNG ODER UMKEHRUNG DER HAUTALTERUNG DURCH ACTIN-SEQUESTRIERENDE PEPTIDE
INHIBITION OU INVERSION DU VIEILLISSEMENT DE LA PEAU AU MOYEN DE PEPTIDES SEQUESTRANT L'ACTINE

(30) Priority: 02.11.2000 US 244901 P
(43) Date of publication of application: 20.08.2003
(73) Proprietor: Regenerx Biopharmaceuticals Inc., Bethesda, MD 20817 (US)
(72) Inventor: GOLDSTEIN, Allan, L., Washington, DC 20037 (US)
(74) Representative: Kaminski Harmann
(86) International application number: PCT/US2001/042900
(87) International publication number: WO 2002/036143

(56) References cited:
- WO-A1-00/06190
- SIMENEL CATHERINE ET AL: "Structural requirements for thymosin beta4 in its contact with actin: An NMR-analysis of thymosin beta4 mutants in solution and correlation with their biological activity" EUROPEAN JOURNAL OF BIOCHEMISTRY, vol. 267, no. 12, June 2000 (2000-06), pages 3530-3538, XP002289476 ISSN: 0014-2956

## Description

### BACKGROUND OF THE INVENTION

### Field of the Invention

The present invention relates to the field of inhibiting or reversing skin aging.

### DESCRIPTION OF THE BACKGROUND ART

The phenomenon called skin "aging" may occur not only with advancing age, but due to other degenerative changes and environmental factors. Skin aging results from deleterious changes in the physiological, biochemical and immunological properties of the skin. Such changes include thinning of the skin, loss of elasticity, alteration in polymerized actin ratios and turnover of polymerized actin, decrease in collagen and other matrix proteins, changes in vasculature which decrease capacity to repair DNA damage, increased propensity for skin cancers such as squamous cell carcinoma, and increased risk of infection.

Numerous pharmaceutical, nutriceutical or cosmeceutical formulations have been proposed to reduce or reverse skin aging or the appearance of skin aging. In addition, chemical peels, phototherapies and various forms of plastic surgery have been proposed.

There remains a need in the art for improved methods and compositions for inhibiting or reversing skin aging.

### SUMMARY OF THE INVENTION

The present invention relates to a use of an effective amount of a skin degeneration-inhibiting polypeptide comprising amino acid sequence LKKTET or a conservative variant thereof having skin degeneration-inhibiting activity, for the manufacture of a composition for promoting reversal of or inhibiting skin degeneration associated with skin aging.

### DETAILED DESCRIPTION OF THE INVENTION

The present invention is based on a discovery that actin-sequestering peptides such as thymosin 134 (Tβ4) and other actin-sequestering peptides containing amino acid sequence LKKTET or conservative variants thereof, promote reversal of or inhibit skin degeneration associated with skin aging.

Thymosin β4 was initially identified as a protein that is up regulated during endothelial cell migration and differentiation *in vitro.* Thymosin β4 was originally isolated from the thymus and is a 43 amino acid, 4.9 kDa ubiquitous polypeptide identified in a variety of tissues. Several roles have been ascribed to this protein including a role in a endothelial cell differentiation and migration, T cell differentiation, actin sequestration and vascularization.

In accordance with one embodiment, the invention is a use of an effective amount of an agent that stimulates production of a skin degeneration-inhibiting polypeptide comprising amino acid sequence LKKTET, or a conservative variant thereof having skin degeneration-inhibiting activity, preferably Thymosin β4, an isoform of Thymosin β4, oxidized Thymosin β4 or an antagonist of Thymosin β4, for the manufacture of a composition for promoting reversal of or inhibiting skin degradation associated with skin aging.

The present invention promotes skin condition improvements selected from the group consisting of an increase in skin elasticity, size reduction of an area of age-related skin darkening (age spots), lightening of an area of age-related skin darkening, and combinations thereof.

Compositions which may be used in accordance with the present invention include Thymosin β4 (Tβ4), Tβ4 isoforms, oxidized Tβ4, polypeptides comprising the amino acid sequence LKKTET or conservative variants thereof having skin degeneration-inhibiting activity. WO 00/06190 discloses isoforms of Tβ4 which may be useful in accordance with the present invention as well as amino acid sequence LKKTET and conservative variants thereof having skin degeneration-inhibiting activity, which may be utilized with the present invention. WO 99/49883 discloses oxidized Thymosin β4 which may be utilized in accordance with the present invention. Although the present invention is described primarily hereinafter with respect to Tβ4 and Tβ4 isoforms, it is to be understood that the following description is intended to be equally applicable to amino acid sequence LKKTET, conservative variants thereof having skin degeneration-inhibiting activity, as well as oxidized Thymosin β4.

In one embodiment, the invention relates to a use of a skin degeneration-inhibiting effective amount of Tβ4 or a Tβ4 isoform for the manufacture of a composition for inhibiting or reversing aging of skin in a subject , wherein the composition may be for topical or systemic administration. Examples of topical administration include, for example, contacting the skin with a lotion, salve, gel, cream, paste, spray, suspension, dispersion, hydrogel, ointment, or oil comprising Tβ4. Systemic administration includes, for example, intravenous, intraperitoneal, intramuscular injections of a composition containing Tβ4 or a Tβ4 isoform. A subject may be any mammal, preferably human.

A composition in accordance with the present invention can be administered daily, every other day, etc., with a single application or multiple applications per day of administration, such as applications 2,3,4 or more times per day of administration.

Tβ4 isoforms have been identified and have about 70%, or about 75%, or about 80% or more homology to the known amino acid sequence of Tβ4. Such isoforms include, for example, tβ4^{ala}, Tβ9, Tβ10, Tβ11, Tβ12, Tβ13, Tβ14 and Tβ15. Similar to Tβ4, the Tβ10 and Tβ15 isoforms have been shown to sequester actin. Tβ4, Tβ10 and Tβ15, as well as these other isoforms share an amino acid sequence, LKKTET, that appears to be involved in mediating actin sequestration or binding. Although not wishing to be bound to any particular theory, the activity of Tβ4 isoforms may be due, in part, to the ability to polymerize actin. For example, Tβ4 can modulate actin polymerization in skin (e.g. β-thymosins appear to depolymerize F-actin by sequestering free G-actin). Tβ4's ability to modulate actin polymerization may therefore be due to all, or in part, its ability to bind to or sequester actin via the LKKTET sequence. Thus, as with Tβ4, other proteins which bind or sequester actin, or modulate actin polymerization, including Tβ4 isoforms having the amino acid sequence LKKTET, are likely to reduce skin aging, alone or in a combination with Tβ4, as set forth herein.

Thus, it is specifically contemplated that known Tβ4 isoforms, such as Tβ4^{ala}, Tβ9, Tβ10, Tβ11, Tβ12, Tβ13, Tβ14 and Tβ15, as well as Tβ4 isoforms not yet identified, will be useful in the invention. The invention therefore further provides pharmaceutical compositions comprising Tβ4, as well as Tβ4 isoforms Tβ4^{ala}, Tβ9, Tβ10, Tβ11, Tβ12, Tβ13, Tβ14 and Tβ15, and a pharmaceutical acceptable carrier.

In addition, other proteins having actin sequestering or binding capability, or that can mobilize actin or modulate actin polymerization, as demonstrated in an appropriate sequestering, binding, mobilization or polymerization assay, or identified by the presence of an amino acid sequence that mediates actin binding, such as LKKTET, for example, can similarly be employed in the invention. Such proteins include gelsolin, vitamin D binding protein (DBP), profilin, cofilin, depactin, Dnasel, vilin, fragmin, severin, capping protein, β-actinin and acumentin, for example.
The invention further provides pharmaceutical compositions comprising gelsolin, vitamin D binding protein (DBP), profilin, cofilin, depactin, Dnasel, vilin, fragmin, severin, capping protein, p-actinin and acumentin as set forth herein. Thus, the invention includes the use of a skin aging reducing polypeptide comprising the amino acid sequence LKKTET and conservative variants thereof.

As used herein, the term "conservative variant" or grammatical variations thereof denotes the replacement of an amino acid residue by another, biologically similar residue. Examples of conservative variations include the replacement of a hydrophobic residue such as isoleucine, valine, leucine or methionine for another, the replacement of a polar residue for another, such as the substitution of arginine for lysine, glutamic for aspartic acids, or glutamin for asparagine, and the like.

Tβ4 has been localized to a number of tissue and cell types and thus, agents which stimulate the production of Tβ4 can be added to or comprise a composition to effect Tβ4 production from a tissue and/or a cell. Such agents include members of the family of growth factors, such as insulin-like growth factor (IGF-1), platelet derived growth factor (PDGF), epidermal growth factor (EGF), transforming growth factor beta (TGF-β), basic fibroblast growth factor (bFGF), thymosin α1 (Tα1) and vascular endothelial growth factor (VEGF). More preferably, the agent is transforming growth factor beta (TGF-β) or other members of the TGF-β superfamily. Tβ4 compositions of the invention may reduce skin aging by effectuating growth of the connective tissue through extracellular matrix deposition, cellular migration and vascularization of the skin.

Additionally, agents that assist or stimulate skin aging reduction may be added to a composition along with Tβ4 or a Tβ4 isoform. Such agents include angiogenic agents, growth factors, agents that direct differentiation of cells, agents that promote migration of cells and agents that stimulate the provision of extracellular matrix material in the skin. For example, and not by way of limitation, Tβ4 or a Tβ4 isoform alone or in combination can be added in combination with any one or more of the following agents: VEGF, KGF, FGF, PDGF, TGFβ, IGF-1, IGF-2, IL-1, prothymosin α and thymosin α1 in an effective amount.

The invention also includes a pharmaceutical composition comprising a therapeutically effective amount of Tβ4 or a Tβ4 isoform in a pharmaceutically acceptable carrier. Such carriers include those listed above with reference to parenteral administration.

The actual dosage or reagent, formulation or composition that inhibits or promotes reversal of skin aging may depend on many factors, including the size and health of a subject. However, persons of ordinary skill in the art can use teachings describing the methods and techniques for determining clinical dosages as disclosed in WO 00/06190, *supra,* and the references cited therein, to determine the appropriate dosage to use.

Suitable topical formulations include Tβ4 or a Tβ4 isoform at a concentration within the range of about 0.001-10% by weight, more preferably within the range of about 0.01 - 0.1% by weight, most preferably about 0.05% by weight.

The therapeutic approaches described herein involve various routes of administration or delivery of reagents or compositions comprising the Tβ4 or other compounds of the invention, including any conventional administration techniques (for example, but not limited to, topical administration, local injection, inhalation, or systemic administration), to a subject. The compositions containing Tβ4 or other compounds of the invention may be formulated into pharmaceutical compositions by admixture with pharmaceutical acceptable non-toxic excipients or carriers.

While not be bound to any particular theory, it is believed that the present invention may promote reversal of or inhibit skin degeneration associated with skin aging by inducing terminal deoxynucleotidyl transferase (a non-template directed DNA polymerase), to decrease the levels of one or more inflammatory cytokines, and to act as a chemotactic factor for endothelial cells, and thereby inhibit or promote reversal of degenerative changes in skin brought about by aging or other degenerative or environmental factors.

The invention is further illustrated by the following non-limiting example.

### Example 1

A 0.05% by weight Thymosin β4 formulation was prepared, *i.e*., 50 mg Thymosin β4 per 100 gm gel, by first dissolving Thymosin β4 in water and thoroughly mixing the preparation in a standard pharmaceutical grade gel formulation. A volunteer with a dark 1 cm age spot on the dorsal region of the hand below the middle knuckle was treated. The 0.05% by weight Thymosin β4 gel was applied to a 5 x 5 cm region encompassing the age spot, twice daily for 28 days. Within seven days the age spot began to fade and within 14 days, the age spot began to noticeably decrease in size. At the end of the 28 day period, the age spot had faded significantly and the diameter of the spot decreased by over 50%. Additionally, the skin in the treated area became smoother and appeared to have increased elasticity. The volunteer was subsequently observed for four weeks, and the changes observed during treatment persisted.

## Claims

1. Use of an effective amount of a skin degeneration-inhibiting polypeptide comprising amino acid sequence LKKTET, or a conservative variant of said amino acid sequence having skin degeneration-inhibiting activity, for the manufacture of a composition for promoting reversal of or inhibiting skin degeneration associated with skin aging.

2. Use according to claim 1, for promoting a skin condition improvement selected from the group consisting of an increase in skin elasticity, size reduction of an area of age-related skin darkening, lightening of an area of age-related skin darkening, and combinations thereof.

3. Use according to claim 1, wherein said polypeptide comprises Thymosin β4 (Tβ4), an isoform of Tβ4 or oxidized Tβ4.

4. Use according to claim 3 together with an effective amount of at least one agent that assists or stimulates skin aging reduction, wherein said agent is selected from the group consisting of vascular endothelial growth factor (VEGF), keratinocyte growth factor (KGF), fibroblast growth factor (FGF),
platelet derived growth factor (PDGF), transforming growth factor beta (TGF-β), insulin-like growth factor 1 (IGF-1), insulin-like growth factor 2 (IGF-2), interleukin 1 (IL-1), prothymosin α and thymosin α1 (Tα1).

5. Use according to claim 4, wherein said growth factor is TGF-β.

6. Use according to claim 1, wherein said composition is for systemic administration.

7. Use according to claim 1, wherein said composition is for topical administration.

8. Use according to claim 7, wherein said composition is in the form of a gel, creme, paste, lotion, spray, suspension, dispersion, salve, hydrogel or ointment formulation.

9. Use according to claim 1, wherein said polypeptide is recombinant or synthetic.

10. Use of an effective amount of an agent that stimulates production of a skin degeneration-inhibiting polypeptide comprising amino acid sequence LKKTET, for the manufacture of a composition for promoting reversal of or inhibiting skin degeneration associated with skin aging, wherein said polypeptide is Thymosin β4 (Tβ4) and wherein said agent is a growth factor selected from the group consisting of insulin-like growth factor (IGF-1), platelet derived growth factor (PDGF), epidermal growth factor (EGF), transforming growth factor beta (TGF-β), basic fibroblast growth factor (bFGF), thymosin α1 (Tα1) and vascular endothelial growth factor (VEGF).

## Patentansprüche

1. Verwendung einer wirksamen Menge eines die Hautdegeneration hemmenden Polypeptids umfassend die Aminosäuresequenz LKKTET oder eine konservative Variante dieser Aminosäuresequenz mit einer die Hautdegeneration hemmenden Aktivität, zur Herstellung einer Zusammensetzung zur Förderung der Umkehrung oder Hemmung der mit einer Hautalterung assoziierten Hautdegeneration.

2. Verwendung gemäß Anspruch 1 zur Förderung einer Verbesserung der Hautbeschaffenheit, ausgewählt aus der Gruppe bestehend aus einer Steigerung der Hautelastizität, Verringerung der Größe eines Bereichs von altersbedingt dunkel verfärbter Haut, Aufhellung eines Bereichs von altersbedingt dunkel verfärbter Haut und Kombinationen davon.

3. Verwendung gemäß Anspruch 1, wobei das Polypeptid Thymosin β4 (Tβ4), eine Isoform von Tβ4 oder oxidiertes Tβ4 umfasst.

4. Verwendung gemäß Anspruch 3 zusammen mit einer wirksamen Menge von mindestens einem Agens, das die Reduktion der Hautalterung unterstützt oder stimuliert, wobei das Agens ausgewählt ist aus der Gruppe bestehend aus vaskulärem endothelialem Wachstumsfaktor (VEGF), Keratinozyten-Wachstumsfaktor (KGF), Fibroblasten-Wachstumsfaktor (FGF), aus Plättchen abgeleitetem Wachstumsfaktor (PDGF), transformierendem Wachstumsfaktor (TGF-β), insulinähnlichem Wachstumsfaktor 1 (IGF-1), insulinähnlichem Wachstumsfaktor 2 (IGF-2), Interleukin 1 (IL-1), Prothymosin α und Thymosin α1 (Tα1).

5. Verwendung gemäß Anspruch 4, wobei der Wachstumsfaktor TGF-β ist.

6. Verwendung gemäß Anspruch 1, wobei die Zusammensetzung zur systemischen Verabreichung vorgesehen ist.

7. Verwendung gemäß Anspruch 1, wobei die Zusammensetzung zur topischen Verabreichung vorgesehen ist.

8. Verwendung gemäß Anspruch 7, wobei die Zusammensetzung in Form eines Gels, einer Creme, einer Paste, einer Lotion, eines Sprays, einer Suspension, einer Dispersion, eines Balsams, eines Hydrogels oder einer Salbe formuliert ist.

9. Verwendung gemäß Anspruch 1, wobei das Polypeptid rekombinant oder synthetisch ist.

10. Verwendung einer wirksamen Menge eines Agens, das die Produktion eines die Hautdegeneration hemmenden Polypeptids umfassend die Aminosäuresequenz LKKTET stimuliert, zur Herstellung einer Zusammensetzung zur Förderung der Umkehrung oder der Hemmung einer mit der Hautalterung assoziierten Hautdegeneration, wobei das Polypeptid Thymosin β4 (Tβ4) ist und wobei das Agens ein Wachstumsfaktor ist, ausgewählt aus der Gruppe bestehend aus insulinähnlichem Wachstumsfaktor (IGF-1), aus Blutplättchen abgeleitetem Wachstumsfaktor (PDGF), epidermalem Wachstumsfaktor (EGF), transformierendem Wachstumsfaktor beta (TGF-β), basischem Fibroblasten-Wachstumsfaktor (bFGF), Thymosin α1 (Tα1) und vaskulärem endothelialem Wachstumsfaktor (VEGF).

## Revendications

1. Utilisation d'une quantité efficace d'un polypeptide inhibant la dégénérescence de la peau, comprenant une séquence d'acide aminé LKKTET, ou un variant conservatif de ladite séquence d'acide aminé présentant une activité inhibant la dégénérescence de la peau, pour la fabrication d'une composition devant favoriser l'inversion ou l'inhibition de la dégénérescence de la peau associée au vieillissement de la peau.

2. Utilisation selon la revendication 1, pour favoriser une amélioration de l'état de la peau, sélectionné dans le groupe composé d'une augmentation de l'élasticité de la peau, une réduction de taille d'une zone de brunissement de la peau liée à l'âge, éclaircissement d'une zone de brunissement de la peau liée à l'âge, et des combinaison de celles-ci.

3. Utilisation selon la revendication 1, dans laquelle ledit polypeptide comprend de la thymosine β4 (Tβ4), un isoform de la Tβ4, ou de la Tβ4 oxydée.

4. Utilisation selon la revendication 3, conjointement avec une quantité efficace d'au moins un agent assistant ou stimulant la réduction du vieillissement de la peau, dans laquelle ledit agent est sélectionné dans le groupe composé d'un facteur de croissante endothélial vasculaire (VEGF), d'un facteur de croissance des kéranocytes (KGF) ; d'un facteur de croissance des fibroblastes (FGF), d'un facteur de croissance dérivé des plaquettes (PDGF), d'un facteur de croissance transformant beta (TGF-β), d'un facteur de croissance 1 analogue à l'insuline (IGF-1), d'un facteur de croissance 2 analogue à l'insuline (IGF-2), d'une interleukine 1 (IL-1), d'une prothymosine α et d'une thymosine α1 (Tα1).

5. Utilisation selon la revendication 4, dans laquelle ledit facteur de croissance est le TGF-β.

6. Utilisation selon la revendication 1, dans laquelle ladite composition est prévu pour une administration systémique.

7. Utilisation selon la revendication 1, dans laquelle ladite composition est prévu pour une administration tropique.

8. Utilisation selon la revendication 7, dans laquelle ladite composition se présente sous la forme d'un gel, crème, pâte, lotion, vaporisation-pulvérisation, suspension, dispersion, pommade, hydrogel ou formulation d'onguent.

9. Utilisation selon la revendication 1, dans laquelle ledit polypeptide est recombinant ou synthétique.

10. Utilisation d'une quantité efficace d'un agent stimulant la production d'un polypeptide inhibant la dégénérescence de la peau, comprenant une séquence d'acide aminé LKKTET, pour la fabrication d'une composition devant favoriser l'inversion ou l'inhibition de la dégénérescence de la peau associée au vieillissement de la peau, dans laquelle ledit polypeptide est la thymosine β4 (Tβ4) et dans laquelle ledit agent est un facteur de croissance sélectionné dans le groupe composé du facteur de croissance analogue à l'insuline (IGF-1), du facteur de croissance dérivé des plaquettes (PDGF), du facteur de croissance épidermique (EGF), du facteur de croissance transformant beta (TGF-β) , du facteur de croissance basique des fibroblastes (FGF), de la thymosine α1 (Tα1) et du facteur de croissance endothélial vasculaire (VEGF).


### SEQUENCE LISTING

<110> REGENERX BIOPHARMACEUTICALS, INC.
<120> INHIBITION OR REVERSAL OF SKIN AGING BY ACTIN SEQUESTERING PEPTIDES
<130> BYP-5719-EP
<140> EP 01981902.8
   <141> 2001-11-02
<150> US 60/244,901
   <151> 2000-11-02
<150> PCT/US01/42900
   <151> 2001-11-02
<160> 1
<170> PatentIn version 3.1
<210> 1
   <211> 6
   <212> PRT
   <213> Artificial sequence
<220>
   <223> Amino acid sequence present in thymosin β4 (Tβ4) and other actin-sequestering peptides
<400> 1
   Leu Lys Lys Thr Glu Thr
   1               5